# EUROPEAN PATENT SPECIFICATION

(11) **EP 1 113 819 B1**
(45) Date of publication and mention of the grant of the patent: **23.02.2011**
(21) Application number: 99948261.5
(22) Date of filing: 17.09.1999
(51) Int. Cl.: A61K 47/48

(54) **ANTIBODY DIRECTED ENZYME PRODRUG THERAPY (ADEPT) WITH GLUCURONIDASE**
ANTIKÖRPER VERWIESENE ENZYMPRODRUGTHERAPIE MIT GLUKURONIDASE
PROCEDES ET COMPOSITIONS PERMETTANT D'AUGMENTER LA TOXICITE SPECIFIQUE CIBLE D'UN MEDICAMENT CHIMIOTHERAPIQUE

(30) Priority: 18.09.1998 US 101039 P
(43) Date of publication of application: 11.07.2001
(73) Proprietor: Immunomedics, Inc., Morris Plains, NJ 07950 (US)
(72) Inventor: GRIFFITHS, Gary, L., Morristown, NJ 07960 (US); HANSEN, Hans, J., Slidell, LA 70458 (US)
(74) Representative: Bohmann, Armin K.
(86) International application number: PCT/US1999/021308
(87) International publication number: WO 2000/016808

(56) References cited:
- EP-A- 0 501 215
- WO-A-91/09134
- WO-A-96/20011
- WO-A-97/41898
- WO-A-99/42593
- WO-A-99/66951
- US-A- 5 851 527
- LEU Y L ET AL: "Design and synthesis of water-soluble glucuronide derivatives of camptothecin for cancer prodrug monotherapy and antibody-directed enzyme prodrug therapy ( ADEPT )." JOURNAL OF MEDICINAL CHEMISTRY, (1999 SEP 9) 42 (18) 3623-8., XP000867705
- DANKS M K ET AL: "Comparison of activation of CPT - 11 by rabbit and human carboxylesterases for use in enzyme/prodrug therapy." CLINICAL CANCER RESEARCH, (1999 APR) 5 (4) 917-24., XP000867715
- TAKAYAMA H ET AL: "Synthesis of a new class of camptothecin derivatives, the long-chain fatty acid esters of 10-hydroxycamptothecin, as a potent prodrug candidate, and their in vitro metabolic conversion by carboxylesterases" BIOORGANIC & MEDICINAL CHEMISTRY LETTERS,GB,OXFORD, vol. 8, no. 5, 3 March 1998 (1998-03-03), pages 415-418, XP004136875 ISSN: 0960-894X
- MELTON ET AL: "Antibody-directed enzyme prodrug therapy (ADEPT). Review article" DRUGS OF THE FUTURE,ES,BARCELONA, vol. 21, no. 2, 1 January 1996 (1996-01-01), pages 167-181, XP002080157 ISSN: 0377-8282
- HAY M.P. ET AL: "Antibody-directed enzyme-prodrug therapy ( ADEPT )." DRUGS OF THE FUTURE, (1996) 21/9 (917-931)., XP000874404
- ROFFLER ET AL: "Anti-neoplastic glucoronide prodrug treatment of human tumor cells targeted with a monoclonal antibody-enzyme conjugate" BIOCHEM. PHARMACOL., vol. 42, 31 December 1991 (1991-12-31), page 2062, XP000874496
- HAISMA ET AL: "A monoclonal antibody-b-glucuronide conjugate as activator of the prodrug epirubicin-glucuronide for specific treatment of cancer" vol. 66, page 474, XP000882039

## Description

### BACKGROUND OF THE INVENTION

The present invention relates to a targeting composition for use in a method for increasing the target-specific toxicity of a chemotherapy drug by pretargeting an enzyme to a mammalian target site and administering a cytotoxic drug known to act at the target site, or a prodrug thereof, which drug is also detoxified to form an intermediate of lower toxicity using the mammal's ordinary metabolic processes, whereby the detoxified intermediate is reconverted to its more toxic form by the pretargeted enzyme and thus has enhanced cytotoxicity at the target site. Where a prodrug is used, a further improvement is achieved by targeting a second enzyme to the target site that converts the prodrug to the active drug. The present invention also relates to a kit comprising a cytotoxic drug or a prodrug of a cytotoxic drug and a targeting composition.

It is a continuing aim of chemotherapy to deliver a higher total dose of chemotherapeutic to a tumor target, and/or lower doses to sensitive non-target tissues. Direct attachment of drugs to specific targeting agents such as monoclonal antibodies (MAbs) has a number of drawbacks, including diminishing a drug's potency and changing the pharmacokinetic properties of the MAb for the worse. Despite this, impressive results have been seen in preclinical animal results using conjugates of MAbs and standard chemotherapy drugs such as doxorubicin (Trail et al., Science 261:212-215,1993 *&* Cancer Res., 57:100-105,1997). A further problem in translating good animal results to the human situation is that in the latter, tumor target uptake of MAbs is often two to four orders of magnitude lower on a percent injected dose per gram basis.

In part to circumvent the above problems a novel approach was tried whereby an antibody enzyme conjugate was administered, followed sometime later by a precursor of an active drug, i.e. a prodrug. The enzyme localized to target by the tumor-specific antibody would act on the prodrug to release active drug at the target. The method has the advantages of not requiring coupling of drug to MAb, and by virtue of targeted enzyme activity the ability to produce large amounts of drug where it is needed. The latter advantage can overcome the issue of low absolute tumor accretion of MAbs in humans.

In a further modification, a binary system for targeting prodrugs using a bispecific monoclonal antibody (bsMAb) was described by Hansen U.S.S.N. 08/445,110 granted as US 5,851,527 (hereinafter, "Hansen '110"). In this system, a bsMAb with an anti-target arm and an anti-enzyme arm is given, followed later by an enzyme, e.g., glucuronidase which is thus targeted to the disease site. Later still, a prodrug, e.g., a. glucuronide prodrug, is administered and the free drug released by the tumor-targeted enzyme. In addition to addressing the issue of low levels of MAb accretion at human tumors, this method has the further advantage of not requiting the coupling of relatively large MAb and enzymes structures, both of whose activities and pharmacokinetic properties can be affected adversely by such conjugations.

One limitation of the bsMAb/prodrug invention as outlined above is the need for a specific antibody directed towed a specific enzyme. Thus, its adoption with different combinations of prodrugs and enzymes would require the preparation of new bsMAbs for each combination.

A need therefore continues to exist for a method for increasing the target-specific toxicity of a chemotherapy drug which is detoxified by normal metabolic processes to form an intermediate of lower toxicity, whereby the detoxified intermediate is reconverted to its more toxic form by the pretargeted enzyme and thus, has enhanced cytotoxicity at the target site.

Melton et al., Drugs of the future, 21, 167, is a review article related to antibody-directed enzyme prodrug therapy (ADEPT).

Hay M.P. et al., Drugs of the future, 21, 917 is also a review article related to antibody-directed enzyme prodrug therapy (ADEPT).

International patent application WO 97/41898 A is related to a targeted combination immunotherpay of cancer.

Roffler et al., Biochem Pharmacol. Vol. 42, pages 2062-2065 is a paper dealing with antineoplastic glucuronide prodrug treatment of human tumor cells targeted with a monoclonal antiody-enzyme conjugate.

Haisma et al., Br. J. Cancer (1992), 66, 474 - 478 is a paper dealing with a monoclonal antibody-ß-glucuronidase conjugate as activator of the prodrug epirubicin-glucuronide for specific treatment of cancer.

### OBJECTS OF THE INVENTION

One object of the present invention is to enable prodrug systems for enhancing chemotherapy of disease, either by using prodrugs as described previously, or by using commercially available drugs, with the knowledge that their detoxification pathways can be harnessed to improve their therapeutic profiles.

Another object of the present invention is to provide agents, compositions and kits useful for treatment of cancer by harnessing a drug's detoxification pathway for improved therapeutic profiles.

Other objects of the present invention will become more readily apparent to those of ordinary skill in the art in light of the following discussion.

### SUMMARY OF THE INVENTlON

These and other objects of the invention are achieved by providing a targeting composition comprising a glucuronidase conjugated to a targeting molecule specifically binding to a target site for use in a method for the treatment of cancer, said method comprising the administration of a cytotoxic drug or a prodrug of a cytotoxic drug selected from the group consisting of CPT-11, etoposide and epirubicin methyl ester, and a kit comprising, in suitable containers,
(a) a cytotoxic drug or a prodrug of a cytotoxic drug, wherein said cytotoxic drug is converted to a detoxified metabolite by the mammal's ordinary metabolic processes; and wherein the cytotoxic drug and the prodrug of a cytotoxic drug is selected from the group consisting of CPT-11, etoposide and epirubicin methyl ester and
(b) a targeting composition comprising a glucuronidase conjugated to a targeting molecule which specifically binds to a target site.

Preferred embodiments may be taken from the attached claims.

### DETAILED DISCUSSION

The prior art discloses the attachment of therapeutic or diagnostic agents directly to an antibody, or to a carrier attached to an antibody. Some of the problems associated with conjugating an agent to the antibody include cross-linking, loss of immunoreactivity, immunogenicity, insufficient loading of the agent on the antibody and inadequate deposition of the agent at the target site. The present invention overcomes these problems by pretargeting an enzyme to a target site and then administering a cytotoxic drug, or a prodrug form converted to its cytotoxic form, which is detoxified by ordinary metabolic processes and then reconverted to the toxic form by the pretargeted enzyme, resulting in a more concentrated cytotoxicity at the target site. , whereby the cytotoxic drug and the prodrug of a cytotoxic drug is selected from the group consisting of CPT-11, etoposide and epirubicin methyl ester

The enzyme pretargeting is accomplished by a described in detail in Hansen '110. enzyme is bound to an antibody that selectively binds to at least one antigen present at the target site. The enzyme is thereby localized at the target site.

The second method of pretargeting an enzyme to a target site as described in Hansen '110 is through a bispecific antibody or antibody fragment (bsMAb), with at least one binding site specific to an antigen at a target site and at least one other binding site specific to an enzyme. The enzyme can be injected in an amount and by a route which enables a sufficient amount of the enzyme to reach the localized antibody and bind to it to form the antibody-enzyme complex *in situ*.

In a third alternative, as described in Hansen '110 a mammal can be given a bsMAb, one arm of which

Optionally, at this time a primary targeting agent clearing composition can be given. This can be, for instance, a secondary antibody reactive with some part of the targeting molecule, i.e., the antibody-enzyme conjugate or the bsMAb, to remove it from circulation. The secondary MAb cane intact or a fragment, mono- or multi-valent and may be further substituted with agents to enhance circulatory clearance such as galactosyl residues. In the latter instance, multiple galactose substitution directs serum-formed complexes of Mab-enzymes conjugate and secondary MAb to receptors on liver hepatocytes. The clearing agent can also be a high MW protein-bearing haptens recognized by one of the arms of the bsMAb. Finally, an entirely separate clearing mechanism can be envisaged. The bsMAb can be further substituted with a hapten (e.g. biotin). Once tumor uptake is maximized, a clearing dose of avidin is given. The latter is rapidly sequestered in the liver, and will remove remaining, non-targeted, biotin-bsMAb from circulation. The clearing mechanism is described here as being given before the enzyme-hapten injection, although it can also be given afterwards.

More preferably, a clearing agent is administered to remove non-targeted enzyme/Mab conjugates from circulation prior to administration of said drug or prodrug. When the enzyme is conjugated to a Mab, the clearing agent can be an anti-idiotypic antibody or anti-idiotypic antibody fragment which is directed to the paratope of the Mab. Clearance of enzyme/Mab conjugates is more effective than clearance of bsMAb, since it limits residual enzyme activity in serum. High residual serum enzyme levels are a limiting factor in the success of targeted immunotherapy.

After the enzyme is pretargeted to the target site, a cytotoxic drug is injected, which is known to act at the target site, or a prodrug form thereof which is converted to the drug *in situ*. The drug is one which is detoxified to form an intermediate of lower toxicity, most commonly a glucuronide, using the mammal's ordinary detoxification processes. The detoxified intermediate, e.g., the glucuronide, is reconverted to its more toxic form by the pretargeted enzyme and thus has enhanced cytotoxicity at the target site, in effect recycling the drug.

In another aspect of the invention, a second enzyme that accelerates conversion of a prodrug into its cytotoxic drug product is also localized at the target site. This can be effected by any of the foregoing three mechanisms for enzyme localization. It is disclosed that it is especially convenient to use a bsMAb whose second arm binds a hapten, and to conjugate the same hapten to each of the two different enzymes. After localization of the bsMAb at the target site, and optional clearance of non-targeted bsMAb, both enzyme conjugates are administered, in appropriate proportions and order, thereby loading the target with both enzymes. When the prodrug reaches the target site, it will be transformed by the enzyme into a product comprising the therapeutic agent. The enzyme can transform many molecules of prodrug to liberate many molecules of drug, which will accrete at the target site. Thus, the enzyme maximizes site-specific generation of the drug from its prodrug form and thereby minimizes systemic side effects. Further amplification of the site-specific activity of the drug is achieved by the second enzyme which converts a naturally detoxified form the drug, e.g., a glucuronide, which is circulating in the bloodstream and which will eventually be excreted, and reconverts it at the target site to its cytotoxic form.

Unless otherwise noted, use of the term "antibody" harem will be understood to include antibody fragments and thus to be equivalent to the term "antibody/fragment" which is used interchangeably therefor in this discussion. Antibodies can be whole immunoglobulin of any class, e.g., IgG, IgM, IgA, IgD, IgE. or hybrid antibodies with dual or multiple antigen or epitope specificities, or fragments. e.g., F(ab')2, F(ab)2, Fab¹, Fab and the like, including hybrid fragments.

Antibodies include antiserum preparations, preferably affinity- purified, having a high immunoreactivity, e.g., a binding constant of at least about 107 l/mole, preferably at least about 1091/mole, a high immumospecificity, e.g., at least about 40%, preferably at least about 60%, more preferably about 70-95%, and a low cross-reactivity with other tissue antigens, e.g., not more than about 30%, preferably not more than about 15% and more preferably not more than about 5%. The antiserum can be affinity purified by conventional procedures, e.g., by binding antigen to a chromatographic column packing. e.g., Sephadex, passing the antiserum through the column, thereby retaining specific antibodies and separating out other immunoglobulins and contaminants, and then recovering purified antibodies by elution with a chaotropic agent, optionally followed by further purification.

Monoclonal antibodies (Mabs) are also suitable for use in connection with the present invention, and are preferred because of their high specificities. They are readily prepared by what are now considered conventional procedures of immunization of mammals with an immunogenic antigen preparation, fusion of immune lymph or spleen cells with an immortal myeloma cell line, and isolation of specific hybridoma clones. More unconventional methods of preparing monoclonal antibodies are not excluded, such as interspecies fusions and genetic engineering manipulations of hypervariable regions, since it is primarily the antigen specificity of the antibodies that affects their utility in the present method.

Antibody fragments can be made by pepsin or papain digestion of whole immunoglobulins by conventional methods such as those disclosed, inter alia, in U.S. Patent 4,331,647.

The target sites can be, but are not limited to, cancers, infectious and parasitic lesions, fibrin clots, myocardial infarctions, atherosclerotic plaque, damaged normal cells, non-cancerous cells and lymphocyte autoreactive clones.

Many antibodies and antibody fragments which specifically bind markers produced by or associated with tumors or infectious lesions, including viral, bacterial, fungal and parasitic infections, and antigens and products associated with such microorganisms have been disclosed, inter alia, in Hansen et al., U.S. Patent 3,927,193 and Goldenberg U.S. Patents 4,331,647, 4,348,376, 4,361,544, 4,468,457, 4,444,744, 4,460,459 and 4,460,561, and in related pending applications U.S. Ser. Nos. 609,607 and 633,999.

Anti-fibrin antibodies are well known in the art. Antibodies that target myocardial infarctions are disclosed in, e.g., Haber, U.S. Patent 4,036,945. Antibodies that target normal tissues or organs are disclosed in, e.g., U.S. Patent No. 4,735,210. Anti-fibrin antibodies are well known in the art, as are antibodies that bind to atherosclerotic plaque and to lymphocyte autoreactive closes.

In general, antibodies can usually be raised to any antigen, using the many conventional techniques now well known in the art. Any targeting antibody to an antigen which is found in sufficient concentration at a site in the body of a mammal which is of therapeutic interest can be used to make the targeting antibody molecule for use in the method of tho invention.

Multispecific antibodies include but are not limited to bispecific antibodies. Bispecific antibodies can be made by a variety of conventional methods, e.g., disulfide cleavage and reformation of mixtures of whole IgG or, preferably, F(ab')2 fragments, fusions of more than one clone to form polyomas that produce immunoglobulins having more than one specificity, and by genetic engineering. The bispecific antibodies can bind to one or more epitopes on the enzyme but should not bind to a site that interferes with enzyme activity. Alternatively, the bispecific will have a non-targeting arm which binds specifically to a low MW hapten, e.g., a DPTA chelate or other convenient hapten.

The recycling enzyme used in the present invention must be capable of transforming a detoxified, normally more sesom-soluble drug, e.g., a glucuronide, to regenerate the drug. Glucuronidases are well known and are suitable recycling enzyme. Other forms of naturally detoxified drugs, such as sulfated or glycosylated (other than glucuronide) molecules can be regenerated at the target site by corresponding enzymes, e.g., sulfatases, glycosylases, and like. Mutated or otherwise optimized forms of these enzymes also are suitable for use. Methods for mutation and optimization of enzymes are wall known in the art. It will be appreciated by those of skill in the art that molecules that function as enzymes, e.g., abzymes (catalytic antibodies), and the like, will also be suitable for use in the invention and are included in the generic term "enzyme" as used herein as a term for a catalytic molecule for cleaving a prodrug or a detoxified drug conjugate. Similarly, the prodrug cleaving enzymes also can be a mutated and/or optimized form of a natural enzyme or an enzyme mimic such as an abzyme or a synthetic or semisynthetic catalytic molecule.

The prodrug cleaving enzyme and the prodrug itself can be one of those described in Hansen '110 or any other appropriate enzyme or enzyme mimic or prodrug that functions in the manner described herein for components of the inventive methods or composition.

The targeting molecule can be labeled with, or conjugated or adapted for conjugation to, a radioisotope or magnetic resonance image enhancing agent, to monitor its clearance from the circulatory system of the mammal and make certain that it has sufficiently localized at the target site, prior to the administration of the drug or prodrug. Alternatively, the targeting molecule can be tagged with a label, e.g., a radiolabel, a fluorescent label or the like, that permits its detection and quantitation in body fluids, e.g., blood and urine, so that targeting and/or clearance can be measured and/or inferred.

Any conventional method of labeling proteins for in vivo use will be generally suitable for labeling the targeting molecule, e.g., those disclosed in Hansen '110 or others well known to the skilled artisan.

The drug or prodrug must be soluble for purposes of administration and transport to the target site and the drug must also be capable of being converted to a detoxified form, e.g., a glucuronide, sulfate or glycoside, by the mammalian body. As used herein, the term "soluble" means soluble in the fluid into which it is administered and by which it is transported to the target site, to a sufficient extent to permit transport of a therapeutically effective amount of the drug or prodrug to such site. Normally, drug administration will be into the bloodstream, by intravenous or intra-arterial infusion, and the drug will need to be soluble in serum and preferably sufficiently hydrophilic to be carried largely by the aqueous phase of serum and diffuse relatively easily through the walls of the blood vessels into interstitial fluid, for cases where such is necessary. Oral forms of a drug or prodrug, or other forms well known in the art, that permit transport of the drug or prodrug to the target site or that permit the prodrug to be converted to a drug which then can be transported to the target site, also will be suitable for use in the invention.

This will be better understood in light of some general examples and some more detailed description of various species.

Certain cytotoxic drugs that are useful for anticancer therapy are relatively insoluble in serum. Some are also quite toxic in unconjugated form and their toxicity is considerably reduced by conversion to prodrugs. Conversion of a relatively poorly soluble drug to a more soluble conjugates, e.g., a glucuronide, an ester of a hydrophilic acid, an amide of a hydrophilic amine, will improve its solubility in the aqueous phase of serum and its ability to pass through venous, arterial or capillary cell walls and reach the interstitial fluid bathing the tumor. Cleavage of the prodrug will deposit the less soluble drug at the target site. Many examples of such prodrug-todrug conversions are disclosed in Hansen '110.

Conversion of certain toxic substances such as aromatic or alicyclic alcohols, thiols, phenols and amines to glucuronides in the liver is the body's method of detoxifying them and making them more easily excreted in the urine. One type of antitumor drug that can be converted to such a substrate is epirubicin, a 4-epimer of doxorubicin (Adriamycin), which is an anthracycline glycoside and has been shown to be a substrate for human beta-D-glucuronidase (Arcamone, Cancer Res., 45:5995, 1995). Other analogues with fewer polar groups would be expected to be more lipophilic and show greater promise for such an approach. Other drugs or toxins with aromatic or alicyclic alcohol, thiol or amine groups would also be candidates for such conjugate formation. These drugs, or other prodrug forms thereof, will be suitable candidates for the site-specific enhancement methods.

Prodrug forms such as carrier polymer-loaded molecules also are suitable for use in the present methods. This latter example allows for the use of a multiply substituted prodrug in the prodrug administration step. As an illustration of the adaptations to be used for other drugs, loading with 5-flourouracil (5-FU) can be effected by oxidizing 5-flourouridine at the carbohydrate portion, e.g., using periodate, reacting this intermediate with an aminodextran, and reductively stabilizing the Schiff base adduct. Cycloheximide can be loaded by direct reaction of its cyclohexanone carbonyl with aminodextran amine groups, followed by reductive stabilization, or by reacting its side chain hydroxyl with an excess of a diisothiocyanate linker and reaction of the isothiocyanate derivative with amines on the aminodextran, or by reaction of the imide nitrogen with e.g., a haloacid or haloester, followed by activation of the resultant carboxyl derivative, e.g., with DCC, and condensation with amines on the aminodextran. The loaded aminodextran is stripped of drug by an amidase pretargeted to the target site. If the drug is detoxified as a glucuronide, the glucuronide of the drug can be cleaved by a glucuronidase which also has been pretargeted to the target site to regenerate and recycle the cytotoxic drug.

Another illustration is provided by the antibiotic mitomycin C and its analogues. This molecule has an amine function and a cyclic imine, either of which can be reacted with an alkylating activating group, e.g., succinimidyloxy iodoacetate or sulfosuccinimidyloxy (4-iodoacetyl) aminobezoate (sulfo-SIAB), the resulting intermediate is then used to alkylate amine groups on an aminodextran. Alternatively, carboxyl groups can be introduced using, e.g., succinic anhydride, then activated, e.g., with DCC. and the activated intermediate coupled as before. Again, a target-localized amidase will liberate the drug, some drug molecules will be detoxified to form glucuronides, and targeted glucuronidase will regenerate the drug to amplify its target-specific activity.

The prodrug CPT-11 (irinotecan) is converted *in vivo* by carboxylesterase to the active metabolite SN-38. Also disclosed is to use a bsMAb targeted against a tumor and a hapten (e.g. DTPA) allowed by injection of a DTPA-carboxyl esterase conjugate. Once a suitable tumor-to-background localization ratio has been achieved, the CPT11 is given and the tumor-localized carboxylesterase serves to convert CPT-11 to SN-38 at the tumor. Since the active SN-38 is poorly soluble it will remain in the vicinity of the tumor and, since it is being generated in the vicinity of the tumor, it is able to exert an effect on adjacent tumor cells that are negative for the antigen being targeted. These are further advantages of the method. Modified forms of carboxylesterase that can be expressed by cells have been described (Patter et al., Cancer Res., 58:2646-2651 and 3627-3632, 1998), and such designed enzymes are within the scope of the invention.

Etoposide is a widely used cancer drug that is detoxified to a major extent by formation of its glucuronide (Hande et al., Cancer Res., 48: 1829-1834, 1988), and can therefore be used within the scope of the invention. Glucuronide conjugated can be prepared from cytotoxic drugs and be injected as therapeutics for tumors pre-targeted with MAb-glucuronidase corrugates (Wang et al., Cancer Res., 52:448-4491, 1992). Accordingly, also disclosed is that such conjugates can also be used with the bsMAb approach disclosed herein here. Designed prodrugs based on derivatives of daunomycin and doxorubicin have been described (Bakina et al., J. Med. Chem., 40:4013-4018, 1997) for use with carboxylesterases and glucuronidases, and these can be used within the scope of the invention. Some other combinations of prodrugs and enzymes are listed. Glucuronide prodrugs of hydroxy derivatives of phenol mustards (Schmidt et al., Bioorg. Med Chem. Lett., 7:1071-1076, 1997) and beta-glucuromidase. Phenol mustards or CPT-11 and carboxypeptidase. Methotrexate-substituted alpha-amino acids and carboxypeptidase A. Beta-lactamase and penicillin or cephalosporin conjugates of drugs such as 6-mercaptopurine and doxorubicin. Alkaline phosphatase and etoposide phosphate.

Many drugs and toxins are known which have a cytotoxic effect on tumor cells or microorganisms that may infect a human and cause a lesion, in addition to the specific illustrations given above. They are to be found in compendia of drugs and toxins, such as the Merck Index and the like. The ability to partially or completely detoxify drug as prodrug according to the invention, while it is in circulation, can reduce systemic side effects of the drug and permit its use when systemic administration of the drug would be unacceptable. For example, MTX and cycloheximide often are too toxic when administered systemically. Administration of the drug as a prodrug which is only converted to the toxic form at the target site by a pretargeted enzyme, together with recycling and reactivation of the detoxified drug at the target site by a pretargeted second enzyme, permits use of a significantly reduced dose which is still effective for therapy at the target site while mitigating systemic toxicity.

The clearance characteristics of drugs can be modulated by certain agents, and the use of such modulating agents within the invention form an additional embodiment. For example, CPT-11 clearance properties have been shown to be modulated by administration of cyclosporin A with the latter reducing the level of biliary clearance of SN-38 and its glucuronide (SN-38G) (Gupta et al., Cancer Res. 56:1309-1314, 1996). In turn, this raised the plasma concentration of SN-38G. This would allow for greater contact with tumor-targeted DTPAglucuronidase in the present invention. Gupta et al. also showed a similar effect when using phenobarbitol (Cancer Chemother. Pharmacol., 39:440-444, 1997). It is disclosed that this agent could also be given along with CPT-11 after pre-targeting DTPA-glucuronidase. In the latter article they also showed that pretreatment of rats with valproic acid (an inhibitor of uridine diphosphate glucuronosyl transferase (UDP-GT) inhibited the formation of SN-38G leading to a 270% AUC for SN-38 from subsequently-administered CPT-11. Thus, use of valproic acid, when pre-targeting DTPA-carboxylesterase to tumor, will also lead to higher levels of SN-38 at the target.

The reagents are conveniently provided as separate injectible preparations for human therapeutic use. The first injectible preparation contains an effective amount of an antibody or antibody fragment conjugated to an enzyme, in a acceptable injection vehicle, preferably phosphate-buffered saline (PBS) at physiological pH and concentration. The second injectible preparation contains an effective amount of a soluble substrate conjugated to at least one therapeutic agent, in a pharmaceutically acceptable injection vehicle, generally similar to that used for the first preparation. The injectible preparations preferably will be sterile, especially if they are intended for use in humans.

The reagents also can be conveniently provided in a therapeutic kit for antibody targeting to a target site, using suitable containers. Either a container holds an effective amount of a targeting molecule, such as an antibody, which specifically binds to the target site, conjugated to an enzyme capable of converting a detoxified drug to its more cytotoxic.

The reagents can be lyophilized for longer shelf stability or provided in the form of solutions, optionally containing conventional preservatives, stabilizers and the like. Other optional components of such kits would normally be container of buffers, labeling reagents, radioisotopes, paramagnetic compounds, second antibody for enhanced clearance, and conventional syringes, columns, vials and the like.

The method in connection with which the targeting composition of the invention is and the kit of the invention may be used, is normally practiced by parenteral injection. The various types of parenteral infections can be, but are not limited to intracavitary (e.g., intraperitoneal), intravenous, intraarterial, intrapleural, intrathecal, intramuscular, intralymphatic and regional intraarterial, intralesional, subcutaneous, catheter perfusion and the like.

For cancer imaging and/or therapy, intravenous, intraarterial or intrapleural administration is normally used for lung, breast, and leukemic tumors. Intraperitoneal administration is advantageous for ovarian tumors. Intrathecal administration is advantageous for brain tumors and leukemia. Subcutaneous administration is advantageous for Hodgkin's disease, lymphoma and breast carcinoma. Catheter perfusion is useful for metastatic lung, breast or germ cell carcinomas of the liver. Intralesional administration is useful for lung and breast lesions.

The enzyme will generally be administered as an aqueous solution in PBS, preferably a sterile solution, especially if it is for use in humans. Advantageously, dosage units of about 50 micrograms to about 5 mg of the enzyme will be administered, either in a single dose or in divided doses, although smaller or larger doses may be indicated in particular cases. It may be necessary to reduce the dosage and/or use antibodies from other species and/or hypoallergenic antibodies, e.g., fragments or hybrid human or primate antibodies, to reduce patient sensitivity, especially for therapy and especially if repeated administrations are indicated for a therapy course or for additional diagnostic procedures. An indication of the need for such cautionary procedures is an increase in human anti-mouse antibody (HAMA) production, which can be determined using an immunoassay.

It usually takes from about 2 to 14 days and preferably 5 to 14 days for IgG antibody to localize at the target site and substantially clear from the circulatory system of the mammal prior to administration of the drug or prodrug. The corresponding localization and clearance time for F(ab)2 and F(ab')2 antibody fragments is from about 2 to 7 days and preferably 4 to 7 days, and from about 1 to 3 days and preferably 3 days for Fab and Fab' antibody fragments. Other antibodies may require different time frames to localize at the target site, and the above time frames may be affected by the presence of the conjugated enzyme. Again, it is noted that labeling the enzymes permits monitoring of localization and clearance.

IgG is normally metabolized in the liver and, to a lesser extent, in the digestive system. F(ab)2 and F(ab')2 are normally metabolized primarily in the kidney, but can also be metabolized in the liver and the digestive system. Fab and Fab' are normally metabolized primarily in the kidney, but can also be metabolized in the liver and the digestive system.

Normally, it will be necessary for at least about 0.0001% of the injected dose of antibody-enzyme conjugate to localize at the target site prior to administration of the substrate-agent conjugate. To the extent that a higher targeting efficiency for this conjugates is achieved, this percentage can be greater, and a reduced dosage can be administered.

It follows that an effective amount of an antibody-enzyme conjugate is that amount sufficient to target the conjugate to the antigen at the target site and thereby bind an amount of the enzyme sufficient to transform enough of the glucuronide to its cytotoxic form to result in accretion of an effective therapeutic amount of the drug at the target site.

The drug may be given in doses, and at times, that can be optimized empirically for a particular combination. It may be given as a single injection or infusion, or may be administered in repeat doses. It is disclosed that it can most preferably be given from one to two hundred hours after the haptenenzyme is administered, and if given in multiple doses, can most preferably be given at intervals from every hour to every three days. The therapeutic drug will be generally administered as an aqueous solution in PBS. Again, this will be a sterile solution if intended for human use. The drug will be administered after a sufficient time has passed for the enzyme to localized at the target site and substantially clear from the circulatory system of the mammal.

An effective amount of a prodrug is that amount sufficient to deliver an effective amount of the drug to the target site. An effective amount of a therapeutic drug is that amount sufficient to treat the target site.

The method in connection with which the targeting composition of the invention is and the kit of the invention may be used can be accomplished by conjugating an effective therapeutic amount of a radioisotope such 88 Y-90 or I-131 (which may be used for both localization and therapy depending on the amount injected) or a drug such as adriamycin for cancer or gentamycin for infection, an immunomodulatory substance such as poly-IC, or a biological toxin such as pokeweed mitogen to the substrate, and depositing a therapeutically effective amount of the agent at the target site.

Dosage units of substrate-agent conjugate will depend upon many factors, each of which can be determined in a relatively straightforward manner, so that optimal dosimetry can be effected. It will be helpful, in the initial dosimetric evaluation, to use a radiolabeled substrate-agent conjugate (if the agent is not itself a radioactive isotope) to determine the degree and rate of deposit of the agent at the target site, and the rate of clearance and biodistribution of non-targeted conjugate. Use of a labeled antibody-enzyme conjugate to estimate the amount of enzyme localized at the target site will also aid in dosimetric analyse

It may be necessary to perform trials for dosimetry, generally using an animal model first, if available, then in a series of patient studies, to optimize the dose of substrate-agent conjugate, as a function of accessibility of the site, mode of administration, turnover number of the enzyme, desired dose of the agent to the site, and rate of clearance of non-targeted conjugate. This will be expected and the techniques for optimization will be within the ordinary skill of the clinician.

Without further elaboration, it is believed that one skilled in the art can, using the preceding description, utilize the present invention to its fullest extent. The following examples are, therefore, to be construed as merely illustrative with the scope of protection being defined by the claims. In the following examples, all temperatures are set forth uncorrected in degrees Celsius; unless otherwise indicated, all parts and percentages are by weight.

### EXAMPLES

### EXAMPLE 1

### Preparation of Antibody-enzyme Conjugates

(A) A substantially monoconjugated enzyme-antibody preparation is prepared by mildly oxidizing the carbohydrate portion of a humanized anti-lymphoma Fab' having a light chain glycosylation site with periodate, then contacting the oxidized Fab' with a dilute solution of glucuronidase (from bovine liver, Worthingon) to produce an antibody-enzyme conjugate, which is then stabilized by borohydride, in the usual manner. The conjugate can be radiolabeled with I-131, by conventional procedures.
(B)) In a similar fashion to the above Part A, the humanized anti-lymphoma Fab' is conjugated to carboxylesterase.

### EXAMPLE 2

### Therapy of Lymphoma

A human patient suffering from lymphoma is infused intravenously with a sterile, pyrogen-free PBS solution containing 5 mg each of the 1-13 1-labeled anti-lymphoma Fab'-glucuronidase and esterase conjugates prepared according to Example 1 hereof. After 3 days, the conjugates are well localized at the target site and substantially cleared from the circulatory system, as determined by gamma scanning.

The patient is then infused intravenously with a sterile, pyrogen-free PBS solution containing 10 mg of epirubicin methyl ester. Subsequent radioimmunodetection shows significant reduction in the lymphoma, compared to a 10 mg dosage of epirubicin prior to targeting with the enzyme conjugates.

### EXAMPLE 3

### Preparation of bispecific antibody (bsMAb)

An IgG x Fab' bsMAb is made in the following manner. Humanized MIN-14 IgG (antiCEA) is treated with sodium periodate to specifically oxidize heavy chain carbohydrate residues. Formed aldehyde groups are reacted with an excess of the commercially available cross-linking agent MBPH {4-(4-N-maleimidophenyl)butyric acid hydrazide; Pierce Chemical Co., Rockford, IL}. Non-reacted MBPH is removed from the modified hMN- 14 MAb by size-exclusion chromatography and the hMN- 14-(maleimide)ₙ intermediate is reacted with a small molar excess of the Fab'-SH fragment of the anti-DTPA MAb {termed 734}, generated from its IgG by standard pepsin digestion and thiol reduction. The desired bsMAb IgG x Fab' moiety is separated from unreacted proteins and non-1:1 conjugates by preparative size-exclusion HPLC. Further purification may be effected by affinity chromatography using a solid-phase-bound DTPA affinity column.

### EXAMPLE 4

### Preparation of clearing agent

Anti-idiotypic MAb to hMN-14, termed W12, is treated over 2 h with a 300-fold molar excess of the imidate from cyanomethyl-2,3,4,6-tetra-O-acetyl-1-thio-β-D-galactopyranoside (CTTG), that is prepared freshly using sodium methoxide. The galactosyl-W12 thus formed is purified by size-exclusion chromatography. Fluorometric analysis of the product in comparison with unmodified MAb shows that approximately 80% of the MAbs lysine residues are substituted with a galactose residue.

### EXAMPLE 5

### Preparation of enzyme-hapten conjugates

The enzyme carboxylesterase (CE) is treated with a five-fold molar excess of DTPA-dianhydride (Sigma Chemical Co., St Louis, MI). After stirring for one hour, the DTPA-CE is purified from free DTPA and aggregated enzyme by preparative size-exclusion HPLC. Approximately 1-2 DTPA units are appended per enzyme. Similarly, glucuronidase is conjugated to DTPA using the dianhydride.

### EXAMPLE 6

### Tumor therapy

A patient with colorectal cancer is given an injection of the bsMAb comprised of an IgGhMN-14 cross-linked with an anti-DTPA Fab' fragment, prepared according to Example 3. After 48 h to allow for maximum accretion in tumors, an amount of galactose-W12, prepared according to Example 4, sufficient to clear nearly all non-target-bound MN-14-IgG x 734-Fab' from the circulation is administered. This amount is between 5 and 15 times the amount of primary bsMAb remaining in circulation at the time-point specified. Three hours after administration of the galactose-W12, a tumor-saturating amount ofa mixture of the DTPA-CE and DTPA glucuronidase conjugates is given, and allowed to clear circulation and normal tissues. Another three hours later, a standard chemotherapy dose of CPT-11 is administered to the patient, generating free SN-38 specifically at the tumor target sites, regenerating free SN-38 at the target sites from the glucuronide, and destroying the tumors.

### EXAMPLE 7

### Tumor therapy

A patient with colorectal cancer is given an injection of the bsMAb as in Example 6. After 48 h to allow for maximum accretion in tumors, an excess amount of same mixture of the DTPA-CE and DTPA-glucuronidase conjugates as in Example 6 is given. Three hours later an amount of galactose-W12 sufficient to clear nearly all non-target-bound M/N-14-IgG x 734-Fab'-DTPA-enzymes from the circulation is administered. This amount is between 5 and 15 times the amount of primary bsMAb complexes remaining in circulation at the time-point specified. Another three hours later, a standard chemotherapy dose of CPT-11 is administered to the patient, generating free SN-38 specifically at the tumor target sites, recycling SN-38 glucuronide at the target site, and destroying said tumors.

## Claims

1. A targeting composition comprising a glucuronidase conjugated to a targeting molecule specifically binding to a mammalian target site for use in a method for the treatment of cancer, said method comprising the administration of a cytotoxic drug or a prodrug of a cytotoxic drug selected from the group consisting of CPT-11, etoposide and epirubicin methyl ester.

2. The targeting composition of claim 1, wherein the target-specific toxicity of the cytotoxic drug or the prodrug is increased, wherein said cytotoxic drug is detoxified by the mammal's ordinary metabolic processes and the enzyme reconverts said detoxified metabolite of said cytotoxic drug to its more toxic form.

3. The targeting composition of claim 1 or 2, wherein said mammal is a human.

4. The targeting composition of any of claims 1 to 3, wherein said cytotoxic drug is administered as a prodrug.

5. The targeting composition of claim 4, wherein said prodrug is the cancer chemotherapy agent CPT-11, which is detoxified by the mammal's ordinary metabolic processes to SN-38-glucuronide, which is then reconverted to SN-38 by the pretargeted glucuronidase.

6. The targeting composition of claim 5, wherein an esterase that cleaves CPT-11 to SN-38 also is pretargeted to said target site.

7. The targeting composition of any of claims 1 to 6, wherein, additionally, a clearing agent is administered to remove non-targeted pretargeting molecules and/or enzymes from said mammal's circulation prior to administration of said drug or prodrug.

8. The targeting composition of claim 7, wherein said clearing agent is an anti-MAb antibody or an anti-idiotype antibody.

9. The targeting composition of claim 7, wherein the glucuronidase is conjugated to a Mab and said clearing agent is an anti-idiotypic antibody or anti-idiotypic antibody fragment which is specific for the paratope of said Mab.

10. A kit comprising, in suitable containers,
(a) a cytotoxic drug or a prodrug of a cytotoxic drug, wherein said cytotoxic drug is converted to a detoxified metabolite by the mammal's ordinary metabolic processes; and wherein the cytotoxic drug and the prodrug of a cytotoxic drug is selected from the group consisting of CPT-11, etoposide and epirubicin methyl ester and
(b) a targeting composition comprising a glucuronidase conjugated to a targeting molecule which specifically binds to a target site.

11. The kit of claim 10, wherein said targeting molecule is a Mab.

12. The kit of claim 10 or 11, which further comprises a clearing agent for said targeting molecule.

13. The kit of any of claims 10 to 12, which further comprises a clearing agent for glucuronidase.

14. The kit of claim 10, wherein the kit is for increasing the target-specific toxicity of a drug.

## Patentansprüche

1. Targetierungszusammensetzung umfassend eine Glucuronidase, die an ein eine Säugetier-Zielstelle spezifisch bindendes Targetierungsmolekül konjugiert ist, zur Verwendung in einem Verfahren für die Behandlung von Krebs, wobei das Verfahren die Verabreichung eines zytotoxischen Wirkstoffs oder eines Wirkstoffpräkursors eines zytotoxischen Wirkstoffs umfasst, der ausgewählt ist aus der Gruppe bestehend aus CPT-1 1, Etoposid und Epirubicin-Methylester.

2. Targetierungszusammensetzung nach Anspruch 1, wobei die zielspezifische Toxizität des zytotoxischen Wirkstoffs oder des Wirkstoffpräkursors erhöht ist, wobei der zytotoxische Wirkstoff durch die üblichen Stoffwechselprozesse des Säugetiers entgiftet wird und das Enzym den entgifteten Metaboliten des zytotoxischen Wirkstoffs in seine toxischere Form rekonvertiert.

3. Targetierungszusammensetzung nach Anspruch 1 oder 2, wobei das Säugetier ein Mensch ist.

4. Targetierungszusammensetzung nach einem der Ansprüche 1 bis 3, wobei der zytotoxische Wirkstoff als ein Wirkstoffpräkursor verabreicht wird.

5. Targetierungszusammensetzung nach Anspruch 4, wobei der Wirkstoffpräkursor das Krebs-Chemotherapieagens CPT-11 ist, das durch die üblichen Stoffwechselprozesse des Säugetiers zu SN-38-Glucuronid entgiftet wird, das dann durch die prätargetierte Glucuronidase zu SN-38 rekonvertiert wird.

6. Targetierungszusammensetzung nach Anspruch 5, wobei eine Esterase, die CPT-11 zu SN-38 spaltet, auch an die Zielstelle prätargetiert ist.

7. Targetierungszusammensetzung nach einem der Ansprüche 1 bis 6, wobei zusätzlich ein Klärmittel verabreicht wird, um nicht-targetierte Prätargetierungsmoleküle und/oder Enzyme vor Verabreichung des Wirkstoffs oder Wirkstoffpräkursors aus dem Kreislauf des Säugetiers zu entfernen.

8. Targetierungszusammensetzung nach Anspruch 7, wobei das Klärmittel ein anti-MAb-Antikörper oder ein anti-Idiotyp-Antikörper ist.

9. Targetierungszusammensetzung nach Anspruch 7, wobei die Glucuronidase an einen Mab konjuguert ist und das Klärmittel ein anti-idiotypischer Antikörper oder ein für das Paratop des Mab spezifisches anti-idiotypisches Antikörperfragment ist.

10. Kit, umfassend in geeigneten Behältern
(a) einen zytotoxischen Wirkstoff oder einen Wirkstoffpräkursor eines zytotoxischen Wirkstoffs, wobei der zytotoxische Wirkstoff durch die üblichen Stoffwechselprozesse des Säugetiers in einen entgifteten Metaboliten konvertiert wird; und wobei der zytotoxische Wirkstoff und der Wirkstoffpräkursor eines zytotoxischen Wirkstoffs ausgewählt ist aus der Gruppe bestehend aus CPT-11, Etoposid und Epirubicin-Methylester und
(b) eine Targetierungszusammensetzung umfassend eine Glucuronidase, die an ein eine Zielstelle spezifisch bindendes Targetierungsmolekül konjugiert ist.

11. Kit nach Anspruch 10, wobei das Targetierungsmolekül ein Mab ist.

12. Kit nach Anspruch 10 oder 11, das weiter ein Klärmittel für das Targetierungsmolekül umfasst.

13. Kit nach einem der Ansprüche 10 bis 12, das weiter ein Klärmittel für Glucuronidase umfasst.

14. Kit nach Anspruch 10, wobei das Kit für das Erhöhen der zielspezifischen Toxizität eines Wirkstoffs ist.

## Revendications

1. Composition de ciblage comprenant une glucuronidase conjuguée à une molécule de ciblage se liant spécifiquement à un site cible de mammifère pour utilisation dans un procédé pour le traitement du cancer, ledit procédé comprenant l'administration d'un médicament cytotoxique ou un promédicament d'un médicament cytotoxique choisi dans le groupe constitué de CPT-11, l'étoposide et l'ester méthylique d'épirubicine.

2. Composition de ciblage de la revendication 1, **caractérisée en ce que** la toxicité spécifique pour la cible du médicament cytotoxique ou du promédicament est augmentée, où ledit médicament cytotoxique est détoxifié par les processus métaboliques ordinaires du mammifère et l'enzyme reconvertit ledit métabolite détoxifié dudit médicament cytotoxique en sa forme plus toxique.

3. Composition de ciblage de la revendication 1 ou 2 **caractérisée en ce que** ledit mammifère est un humain.

4. Composition de ciblage de l'une quelconque des revendications 1 à 3, **caractérisée en ce que** ledit médicament cytotoxique est administré sous la forme d'un promédicament.

5. Composition de ciblage de la revendication 4, **caractérisée en ce que** ledit promédicament est l'agent chimiothérapeutique anticancéreux CPT-11, qui est détoxifié par les processus métaboliques ordinaires du mammifère en SN-38-glucuronide, qui est ensuite reconverti en SN-38 par la glucuronidase préciblée.

6. Composition de ciblage de la revendication 5, **caractérisée en ce qu'**une estérase qui clive CPT-11 en SN-38 est préciblée vers ledit site cible.

7. Composition de ciblage de l'une quelconque des revendications 1 à 6, **caractérisée en ce que**, en outre, un agent de clairance est administré pour éliminer des molécules de préciblage non ciblées et/ou des enzymes de la circulation dudit mammifère avant administration dudit médicament ou promédicament.

8. Composition de ciblage de la revendication 7, **caractérisée en ce que** ledit agent de clairance est un anticorps anti-Mab ou un anticorps anti-idiotype.

9. Composition de ciblage de la revendication 7, **caractérisée en ce que** la glucuronidase est conjuguée à un Mab et ledit agent de clairance est un anticorps anti-idiotypique ou fragment d'anticorps anti-idiotypique qui est spécifique pour le paratope dudit Mab.

10. Kit comprenant, dans des conteneurs adaptés,
(a) un médicament cytotoxique ou un promédicament d'un médicament cytotoxique, **caractérisé en ce que** ledit médicament cytotoxique est converti en métabolite détoxifié par les processus métaboliques ordinaires du mammifère ; et où le médicament cytotoxique et le promédicament d'un médicament cytotoxique est choisi dans le groupe constitué de CPT-11, l'étoposide et l'ester méthylique d'épirubicine et
(b) une composition de ciblage comprenant une glucuronidase conjuguée à une molécule de ciblage qui se lie spécifiquement à un site cible.

11. Trousse de la revendication 10, **caractérisée en ce que** ladite molécule de ciblage est un Mab.

12. Trousse de la revendication 10 ou 11, qui comprend en outre un agent de clairance pour ladite molécule de ciblage.

13. Trousse de l'une quelconque des revendications 10 à 12, qui comprend en outre un agent de clairance pour la glucuronidase.

14. Trousse de la revendication 10, **caractérisée en ce que** la trousse est destinée à augmenter la toxicité spécifique pour la cible d'un médicament.
